# EUROPEAN PATENT SPECIFICATION

(11) **EP 3 437 580 B1**
(45) Date of publication and mention of the grant of the patent: **03.05.2023**
(21) Application number: 17775245.8
(22) Date of filing: 29.03.2017
(51) Int. Cl.: A61B 18/12, A61B 18/14, A61B 18/00, A61B 17/28, A61B 17/29

(54) **FORCEPS-TYPE TREATMENT INSTRUMENT**
ZANGENARTIGES BEHANDLUNGSINSTRUMENT
INSTRUMENT DE TRAITEMENT DE TYPE PINCE

(30) Priority: 31.03.2016 JP 2016071556; 31.03.2016 JP 2016071600
(43) Date of publication of application: 06.02.2019
(73) Proprietor: Zeon Corporation, Tokyo 100-8246 (JP)
(72) Inventor: MINAMITSUJI, Makoto, Tokyo 100-8246 (JP)
(74) Representative: Maiwald GmbH
(86) International application number: PCT/JP2017/012936
(87) International publication number: WO 2017/170711

(56) References cited:
- EP-A1- 2 822 495
- EP-A2- 2 263 586
- WO-A1-2009/003575
- DE-U1-202007 009 165
- JP-A- 2001 259 263
- JP-A- 2003 299 669
- JP-A- 2014 188 160

## Description

### TECHNICAL FIELD

The present invention relates to a forceps type treatment instrument which performs a treatment such as hemostasis by a high-frequency current using, for example, an endoscope.

### BACKGROUND ART

A bipolar hemostat as an example of a forceps type treatment instrument is a device which opens and closes a pair of electrodes (forceps pieces) disposed at a front end of a sheath passing through a treatment instrument guide tube of an endoscope by the operation of an operation part provided at a rear end of the sheath and allowing a high-frequency current to flow to a biological tissue such as a blood vessel while gripping the biological tissue with the high-frequency electrodes so that the tissue in the vicinity of the grip portion is burned and coagulated and hemostasis is performed.

As the bipolar hemostat, for example, a device disclosed in Patent Document 1 is known. In the related art, each of the pair of electrodes (the forceps pieces) includes a shaft hole corresponding to a rotation center and is rotatably and axially supported by a pair of shaft portions of an insulation spacer member for electrically insulating both members from each other. Then, a front end portion of the shaft portion of the insulation spacer member is fitted to a fitting hole of each of a pair of arm portions of a forceps support member provided at the front end of the sheath to be attached thereto. The opening and closing angle (the opening degree) of the pair of electrodes is defined in such a manner that a circular-arc concave groove is formed in an insulation spacer at a contact surface (sliding surface) between the insulation spacer and the electrode, a convex protrusion is formed in the electrode to be loosely fitted to the groove, and the protrusion comes into contact with both end portions of the groove with the rotation of the electrode.

However, according to the related art, the opening and closing angle of the pair of electrodes is defined by the circular-arc concave groove formed in the insulation spacer member at the contact surface (the sliding surface) between the insulation spacer member and the electrode and the convex protrusion formed in the electrode to be loosely fitted to the groove. For this reason, only small protrusion can be formed in structure or dimension and a contact portion between the protrusion portion and the end portion of the groove cannot be formed to be sufficiently large. For that reason, since the protrusion is not stopped by the end portion of the groove and is separated from the groove (the protrusion gets on the outer portion from the end portion of the groove) when a relatively strong rotation force is applied to the electrode, there is concern that the electrode opening and closing operation may be disturbed. When the protrusion repeatedly gets on the outer portion of the groove, there is concern that the end portion of the groove may be partially cut.

For example, when cutting is used to form the protrusion on the side surface (plane) of the electrode, there is a need to cut the other portion as a plane while remaining the protrusion portion at the sliding surface with respect to the insulation spacer member. As a result, since the processing requires much effort, a problem arises in that manufacturing cost increases.

Further, according to the related art, since the shaft portion supporting the electrode of the insulation spacer member and the fitting hole formed in the arm portion of the forceps support member so that the shaft portion is fitted to the fitting hole have a circular cross-section (in a cross-section orthogonal to the axis), there is a case in which the insulation spacer member may rotate with respect to the forceps support member at the time of opening and closing the electrode (particularly, at the time of closing the electrode). As a result, since the posture of the electrode becomes unstable, the posture may be different from the posture that is desired by an operator.

### CITATION LIST

### PATENT DOCUMENT

Patent Document 1: JP 2014-188160 A An electrosurgical forceps with jaws interposed between supporting arms and rotatably supported by a spacer is known from DE 20 2007 009165 U1.

### SUMMARY OF THE INVENTION

### PROBLEM TO BE SOLVED BY THE INVENTION

The present invention has been made in view of such circumstances and an object of the present invention is to provide a forceps type treatment instrument capable of stably opening and closing forceps pieces. Further, another object of the present invention is to provide a forceps type treatment instrument capable of stabilizing a forceps piece opening and closing posture at the time of opening and closing forceps pieces.

### MEANS FOR SOLVING PROBLEM

According to a first aspect of the present invention, there is provided a forceps type treatment instrument including: a forceps support member that includes a first arm portion and a second arm portion attached to a distal end of a sheath and facing each other; a first forceps piece and a second forceps piece which are interposed between the first arm portion and the second arm portion of the forceps support member; and a spacer member that rotatably supports each of the first forceps piece and the second forceps piece, in which the first forceps piece includes a first shaft hole, in which the second forceps piece includes a second shaft hole, in which the spacer member includes a first shaft portion rotatably inserted into the first shaft hole and a second shaft portion rotatably inserted into the second shaft hole, in which the spacer member is provided with a first protrusion portion and a second protrusion portion, in which an inner peripheral surface of the first shaft hole is provided with a first notch portion, in which an inner peripheral surface of the second shaft hole is provided with a second notch portion, in which the first notch portion is provided with a first end portion with which the first protrusion portion comes into contact when the first forceps piece is rotated in a first direction and a second end portion which is the other end facing the first end portion, and in which the second notch portion is provided with a third end portion with which the second protrusion portion comes into contact when the second forceps piece is rotated in a second direction opposite to the first direction and a fourth end portion which is the other end facing the third end portion.

In the forceps type treatment instrument according to the first aspect of the present invention, the spacer member is provided with the protrusion portion (the first protrusion portion, the second protrusion portion) and the inner peripheral surface of the shaft hole of the forceps piece is provided with the notch portion (the first notch portion, the second notch portion) having the end portion (the first end portion, the third end portion) into which the protrusion portion comes into contact. For that reason, since a contact portion between the protrusion portion and the end portion of the notch portion can be set to be sufficiently large, the protrusion portion is not separated from the notch portion even when a relatively strong rotation force is applied to the forceps piece. Further, since the protrusion portion can be reliably stopped by the end portion of the notch portion, it is possible to stably open and close the forceps pieces. Since it is possible to prevent the protrusion portion from being separated from the notch portion, it is possible to reduce the scraping due to the sliding of the notch portion or the protrusion portion, to reduce the damage of the components, and to improve the durability.

In the forceps type treatment instrument according to the first aspect of the present invention, the first protrusion portion may come into contact with the second end portion when the first forceps piece is rotated in the second direction, and the second protrusion portion may come into contact with the fourth end portion when the second forceps piece is rotated in the first direction.

When the protrusion portions (the first protrusion portion, the second protrusion portion) are brought into contact with the end portions (both first and second end portions, both third and fourth end portions) of each of the notch portions (the first notch portion, the second notch portion), the rotatable range or the opening degree of the forceps piece can be defined in both forceps piece opening and closing directions.

In the forceps type treatment instrument according to the first aspect of the present invention, the first protrusion portion may be provided to protrude from an outer surface of the first shaft portion, and the second protrusion portion may be provided to protrude from an outer surface of the second shaft portion.

When the protrusion portion is provided so as to protrude from the outer surface of the shaft portion (the first shaft portion, the second shaft portion) of the spacer, the protrusion portion is reinforced by the shaft portion. For this reason, since it is possible to ensure a sufficient strength without increasing the size of the protrusion portion and to integrally mold the protrusion portion with the spacer, it is possible to decrease a manufacturing cost.

According to a second aspect of the present invention, there is provided a forceps type treatment instrument including: a forceps support member that includes a first arm portion and a second arm portion attached to a distal end of a sheath and facing each other; a first forceps piece and a second forceps piece which are interposed between the first arm portion and the second arm portion of the forceps support member; and a spacer member that rotatably supports each of the first forceps piece and the second forceps piece, in which the first forceps piece includes a first shaft hole, in which the second forceps piece includes a second shaft hole, in which the spacer member includes a first shaft portion rotatably inserted into the first shaft hole and a second shaft portion rotatably inserted into the second shaft hole, in which the first arm portion includes a first fitting hole into which a front end portion of the first shaft portion is fitted, in which the second arm portion includes a second fitting hole into which a front end portion of the second shaft portion is fitted, in which a cutout portion is provided in at least one of the first fitting hole and the front end portion of the first shaft portion, and in which a complementary portion which complements the cutout portion is provided in at least the other one of the first fitting hole and the front end portion of the first shaft portion.

In the forceps type treatment instrument according to the second aspect of the present invention, the cutout portion is provided in at least one of the first fitting hole and the front end portion of the first shaft portion and the complementary portion which complements the cutout portion is provided at the other thereof. For this reason, since the cutout portion and the complementary portion engage with each other in the rotation direction in a state in which the front end portion of the first shaft portion is fitted to the first fitting hole of the first arm portion, it is possible to prevent the rotation of the spacer member with respect to the forceps support member and to stabilize the forceps piece opening and closing posture. That is, when the spacer member rotates with respect to the forceps support member, there is concern that the direction of the distal end of the forceps portion including the pair of forceps pieces rotating with respect to the spacer member to be opened and closed may be inclined with respect to the forceps support member. In the forceps type treatment instrument according to the second aspect of the present invention, since it is possible to reduce such concern, it is possible to provide a forceps type treatment instrument having excellent operability.

In the forceps type treatment instrument according to the second aspect of the present invention, a cutout portion may be provided in at least one of the second fitting hole and the front end portion of the second shaft portion, and a complementary portion which complements the cutout portion may be provided in at least the other one of the second fitting hole and the front end portion of the second shaft portion.

In the forceps type treatment instrument according to the second aspect of the present invention, a cross-sectional shape orthogonal to an axis of at least the first fitting hole and the front end portion of the first shaft portion may be a shape in which at least a part of a circular shape is cutting out.

In the forceps type treatment instrument according to the second aspect of the present invention, an outer peripheral surface of the first shaft portion or an inner peripheral surface of the first shaft hole may be provided with a first protrusion portion, an inner peripheral surface of the first shaft hole or an outer peripheral surface of the first shaft portion may be provided with a first notch portion, an outer peripheral surface of the second shaft portion or an inner peripheral surface of the second shaft hole may be provided with a second protrusion portion, an inner peripheral surface of the second shaft hole or an outer peripheral surface of the second shaft portion may be provided with a second notch portion, the first notch portion may be provided with a first end portion with which the first protrusion portion comes into contact when the first forceps piece rotates in a first direction and a second end portion into which the first protrusion portion comes into contact when the first forceps piece rotates in a second direction opposite to the first direction, and the second notch portion may be provided with a third end portion into which the second protrusion portion comes into contact when the second forceps piece rotates in the second direction and a fourth end portion into which the second protrusion portion comes into contact when the second forceps piece rotates in the first direction.

### BRIEF DESCRIPTION OF DRAWINGS

Fig. 1 is a plan view illustrating an overall configuration of a forceps type treatment instrument of a first embodiment of the present invention;
Fig. 2A is a perspective view illustrating a configuration in the vicinity of an electrode of the forceps type treatment instrument illustrated in Fig. 1;
Fig. 2B is a perspective view illustrating the configuration in the vicinity of the electrode of the forceps type treatment instrument illustrated in Fig. 1 when viewed from a different angle;
Fig. 2C is an exploded perspective view of a kingpin in the vicinity of the electrode illustrated in Fig. 2A;
Fig. 2D is an exploded perspective view of a washer in the vicinity of the electrode illustrated in Fig. 2B;
Fig. 3A is a longitudinal sectional view illustrating a configuration of a second forceps piece of the forceps type treatment instrument illustrated in Fig. 1;
Fig. 3B is a longitudinal sectional view illustrating a configuration of a first forceps piece of the forceps type treatment instrument illustrated in Fig. 1;
Fig. 4A is a perspective view illustrating a configuration of an insulation spacer of the forceps type treatment instrument illustrated in Fig. 1;
Fig. 4B is a right side view of the insulation spacer illustrated in Fig. 4A;
Fig. 4C is a front view of the insulation spacer illustrated in Fig. 4A;
Fig. 4D is a left side view of the insulation spacer illustrated in Fig. 4A;
Fig. 5A is a perspective view illustrating a configuration in the vicinity of an electrode of a forceps type treatment instrument of a second embodiment of the present invention and is an exploded view of a kingpin;
Fig. 5B is a plan view illustrating a configuration in the vicinity of the electrode without the kingpin illustrated in Fig. 5A;
Fig. 5C is a perspective view illustrating the configuration in the vicinity of the electrode of the forceps type treatment instrument illustrated in Fig. 5A when viewed from a different angle and is an exploded view of a washer in the vicinity of the electrode;
Fig. 6A is a longitudinal sectional view illustrating a configuration of a second forceps piece of the forceps type treatment instrument illustrated in Fig. 5A;
Fig. 6B is a longitudinal sectional view illustrating a configuration of a first forceps piece of the forceps type treatment instrument illustrated in Fig. 5A;
Fig. 7A is a perspective view illustrating a configuration of an insulation spacer of the forceps type treatment instrument illustrated in Fig. 5A;
Fig. 7B is a right side view of the insulation spacer illustrated in Fig. 7A;
Fig. 7C is a front view of the insulation spacer illustrated in Fig. 7A;
Fig. 7D is a left side view of the insulation spacer illustrated in Fig. 7A;
Figs. 8(A) to 8(D) are diagrams illustrating a modified example of the forceps type treatment instrument of the second embodiment;
Fig. 9A is a partially exploded perspective view illustrating a configuration in the vicinity of an electrode of a forceps type treatment instrument of a third embodiment of the present invention;
Fig. 9B is a partially exploded perspective view illustrating the vicinity of the electrode of the forceps type treatment instrument illustrated in Fig. 9A when viewed from a different angle;
Fig. 10A is a perspective view illustrating a configuration of an insulation spacer illustrated in Figs. 9A and 9B; and
Fig. 10B is a right side view of the insulation spacer illustrated in Fig. 10A.

### MODE(S) FOR CARRYING OUT THE INVENTION

### First Embodiment

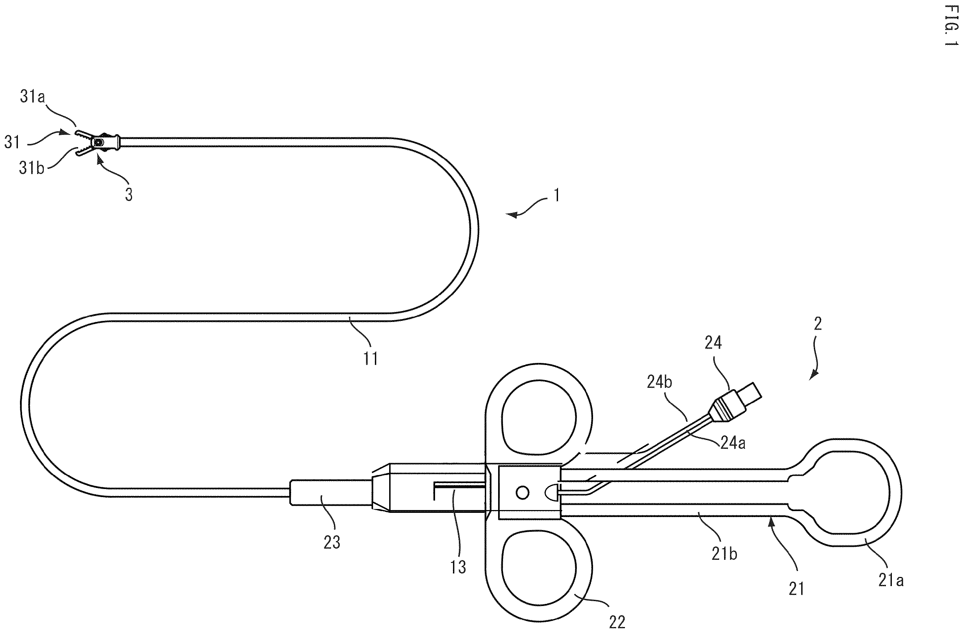

Hereinafter, a bipolar hemostat as a forceps type treatment instrument according to a first embodiment of the present invention will be described in detail with reference to Figs. 1, 2A to 2D, 3A, 3B, and 4A to 4D. However, the present invention is not limited to the bipolar hemostat and can be also applied to a bipolar knife or the like including forceps pieces corresponding to a pair of openable and closable electrodes and a forceps type treatment instrument including forceps pieces not functioning as electrodes.

As illustrated in Fig. 1, the bipolar hemostat of the present embodiment schematically includes a sheath part 1, an operation part 2, and a treatment part 3. The operation part 2 is provided at a proximal end of the sheath part 1 and the treatment part 3 is provided at a distal end of the sheath part 1.

The operation part 2 includes a base 21 and a slider 22 which is attached to the base 21 in a slidable manner, the treatment part 3 includes a forceps portion 31 with a pair of electrodes (a first forceps piece and a second forceps piece) 31a and 31b provided to be openable and closable, and the forceps portion 31 can be opened and closed and rotated by the operation of the operation part 2.

The operation part 2 includes a pair of electric wires (cables) 24a and 24b and plugs 24 respectively provided at base end portions thereof and the electric wires 24a and 24b are electrically connected to a high-frequency power supply (not illustrated) through the plugs 24 so that a high-frequency current is supplied thereto. The electric wire 24a is electrically connected to the first forceps piece 31a and the electric wire 24b is electrically connected to the second forceps piece 31b.

The sheath part 1 includes a tubular outer sheath 11, a tubular inner sheath (not illustrated), and a drive wire 13. The outer sheath 11 is formed as a flexible hollow tube and a tube which is formed of insulation resin is used. In the same way, the inner sheath is formed as a flexible hollow tube and a tube which is formed of insulation resin is used. A resin material of forming the outer sheath 11 and the inner sheath is not particularly limited as long as the insulation material is used and polyethylene, polypropylene, polyvinyl chloride, polyurethane, polyamide, polyester, polycarbonate, polyether sulfone, fluororesin and the like can be used.

The drive wire 13 is inserted through the inner sheath and the inner sheath is inserted through the outer sheath 11. The drive wire 13 is rotatable about the axis and is slidable (slidable along the axial direction) inside the inner sheath.

The drive wire 13 is formed as two flexible and conductive wires and the surfaces of two wires are respectively coated with an insulation coating to be insulated from each other.

The drive wire 13 is a power transmission member that transmits a sliding force for opening and closing the forceps portion 31 and a rotating force for rotating the forceps portion 31 about a sheath axis to adjust the posture thereof.

The operation part 2 schematically includes a base 21, a slider 22, and an end cap 23. The base 21 includes a ring portion 21a and a guide portion 21b with a guide groove. The slider 22 is attached to be slidable along the guide portion 21b of the base 21 in the front and rear direction (the left and right direction of Fig. 1). The end cap 23 is attached to a front end (distal end) of the base 21. The base 21, the slider 22, and the end cap 23 are mainly formed of insulation resin.

The end cap 23 includes an approximately cylindrical space at the inside of the front end portion thereof and a proximal end of the sheath part 1 including the outer sheath 11, the inner sheath (not illustrated), and the drive wire 13 is inserted thereinto through a penetration portion formed at a front end of the end cap 23.

The drive wire 13 can be slid inside the sheath part 1 in the axial direction when the slider 22 of the operation part 2 is slid with respect to the base 21 in the front and rear direction (the left and right direction of Fig. 1) and the drive wire 13 can be rotated about the axis inside the sheath part 1 when the operation part 2 is rotated with respect to the sheath part 1 (the outer sheath 11) .

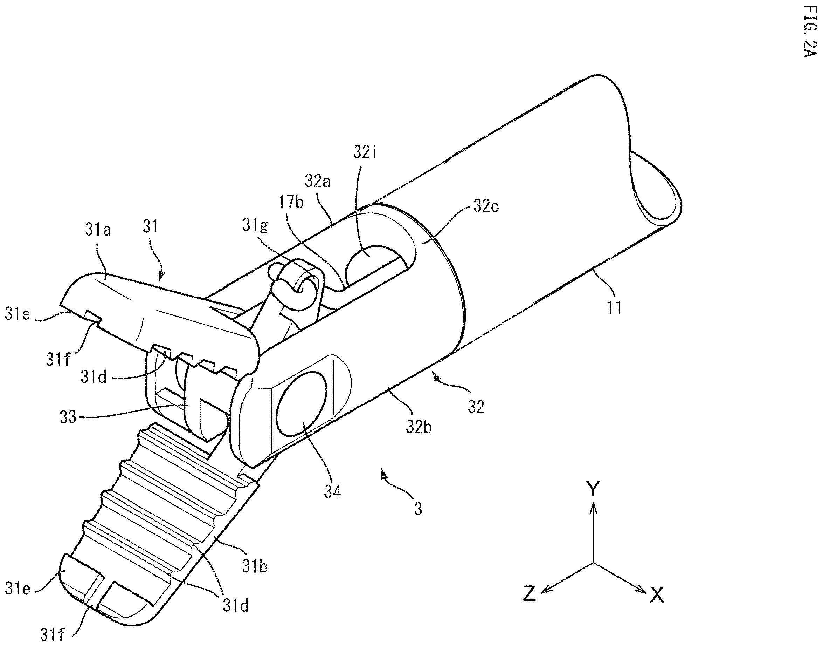

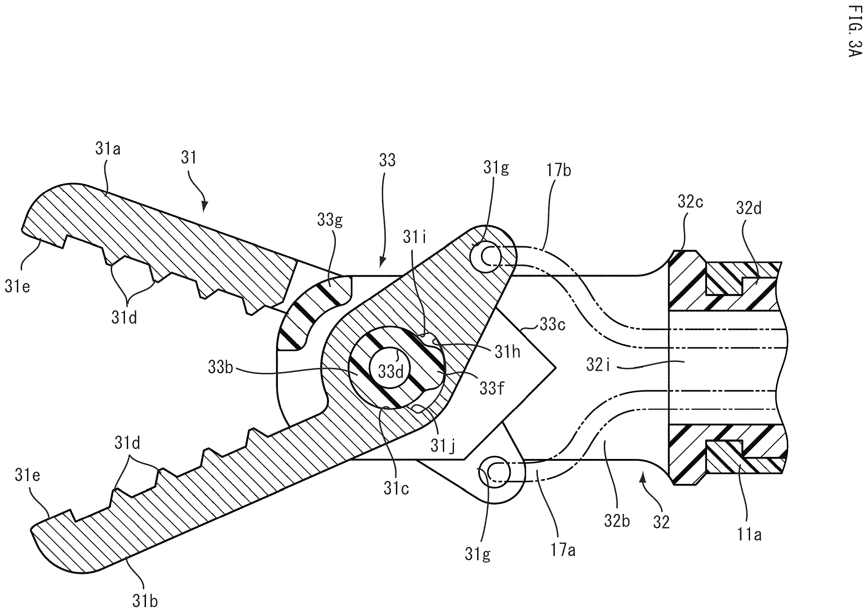

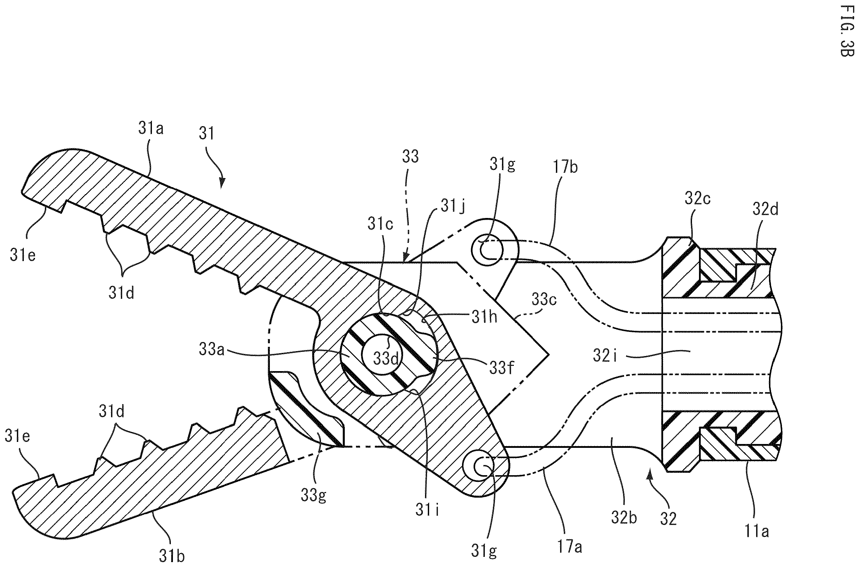

The treatment part 3 schematically includes, as illustrated in Figs. 2A to 2D, 3A, and 3B, the forceps portion 31 including the first forceps piece 31a and the second forceps piece 31b corresponding to a pair of electrodes, a resin frame (forceps support member) 32, an insulation spacer (spacer member) 33, and the like. The resin frame 32 includes a base end portion 32c which includes an approximately cylindrical head portion 32d (see Figs. 3A and 3B) and a pair of arm portions (a first arm portion and a second arm portion) 32a and 32b which protrudes from the base end portion 32c in the Z-axis direction and faces each other in the X-axis direction. Note that, in the drawings, the X axis, the Y axis, and the Z axis are perpendicular to one another.

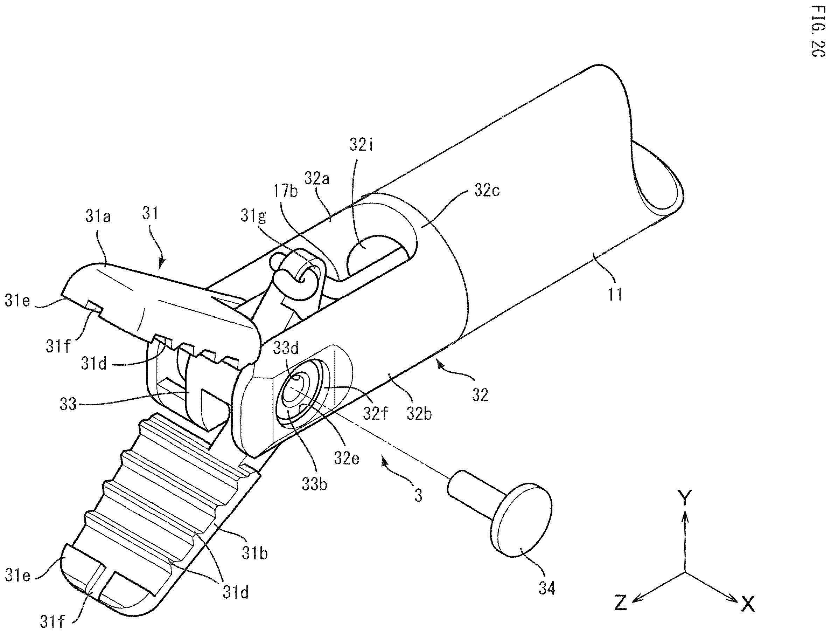

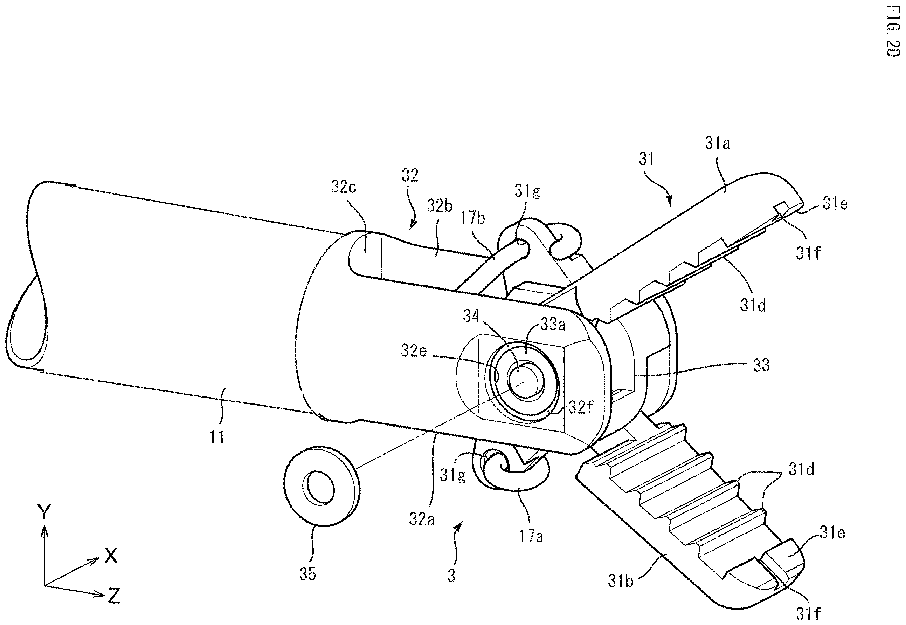

As illustrated in Figs. 2C and 2D, an approximately circular concave portion 32f is formed in the vicinity of the front end portions of the first arm portion 32a and the second arm portion 32b from the outside (non-opposing side). A fitting hole (first fitting hole) 32e is formed in the concave portion 32f of the first arm portion 32a to penetrate the inside and the outside thereof (see Fig. 2D). A fitting hole (second fitting hole) 32e is formed in the concave portion 32f of the second arm portion 32b to penetrate the inside and the outside thereof (see Fig. 2C). The head portion 32d of the resin frame 32 is rotatably attached to the distal end of the outer sheath 11 through a rotation support member (not illustrated) including a fixed member 11a (see Figs. 3A and 3B) provided at the distal end portion of the outer sheath 11.

The base end portion 32c and the head portion 32d of the resin frame 32 are provided with a through-hole 32i and a first wire member 17a and a second wire member 17b which are formed of conductive steel wires and are coated with an insulation coating are disposed to pass through the through-holes 32i. Although not illustrated in the drawings, the proximal ends of the first wire member 17a and the second wire member 17b are respectively fixed to the distal ends of two wires constituting the drive wire 13, the distal end of the first wire member 17a is connected to a through-hole 31g of the first forceps piece 31a, and the distal end of the second wire member 17b is connected to the through-hole 31g of the second forceps piece 31b.

In accordance with the sliding of the drive wire 13, the sliding force is transmitted to the first forceps piece 31a and the second forceps piece 31b through the first wire member 17a and the second wire member 17b so that the first forceps piece 31a and the second forceps piece 31b can be opened and closed. The first forceps piece 31a and the second forceps piece 31b which are located at the opposite sides in the Y-axis direction rotate about the rotation shaft (kingpin 34) following the X axis to move close to the insulation spacer 33 and the forceps portion 31 is closed so that an affected part to be treated can be clamped between the grip portions of the forceps pieces. At the time of opening the forceps portion 31, a reverse operation may be performed.

Note that, the proximal end of the first wire member 17a is fixed to the drive wire 13 while being electrically connected to one of two wires constituting the drive wire 13, so that the first forceps piece 31a is electrically connected to the electric wire 24a (see Fig. 1) through the first wire member 17a and the drive wire 13. Further, the proximal end of the second wire member 17b is fixed to the drive wire 13 while being electrically connected to the other of two wires constituting the drive wire 13. Therefore, the second forceps piece 31b is electrically connected to the electric wire 24b (see Fig. 1) through the second wire member 17b and the drive wire 13.

The fitting holes 32e and 32e of the arm portions 32a and 32b are formed in an approximately circular shape in which the front end portions of the shaft portions 33a and 33b of the insulation spacer 33 described later are fittable thereto.

The first forceps piece 31a and the second forceps piece 31b constituting the forceps portion 31 are rotatably supported while being electrically insulated by the insulation spacer (spacer member) 33 formed of insulation resin (for example, polycarbonate) to have a roughly X-shape and are disposed between the first arm portion 32a and the second arm portion 32b of the resin frame 32 in this state.

The grip portions on the front end sides of the first forceps piece 31a and the second forceps piece 31b are provided with a plurality of (in this example, four) mountain teeth 31d which extend in a direction (hereinafter, referred to as a lateral direction) following the rotation shaft (X axis) of each of the first forceps piece 31a and the second forceps piece 31b in parallel to a direction (hereinafter, referred to as the longitudinal direction) approximately orthogonal to the lateral direction and is also provided with a front end convex tooth 31e. A center portion of the front end convex tooth 31e is provided with a single front end longitudinal groove 31f in the longitudinal direction.

In the first forceps piece 31a and the second forceps piece 31b, as illustrated in Figs. 3A and 3B, an intermediate portion thereof is provided with a shaft hole (a first shaft hole, a second shaft hole) 31c having an approximately circular cross-section and a rear end thereof is provided with a through-hole 31g to which the first wire member 17a or the second wire member 17b is attached.

A part of the inner peripheral surface of the shaft hole 31c of the first forceps piece 31a is provided with a notch portion (first notch portion) 31h which is widened from the axis center outward in the radial direction. The notch portion 31h is provided with an end portion (first end portion) 31i with which a protrusion portion (first protrusion portion) 33f formed in the shaft portion 33a of the insulation spacer 33 comes into contact when the first forceps piece 31a rotates in a first direction (a direction in which the grip portion of the first forceps piece 31a moves close to the grip portion of the second forceps piece 31b, a counter-clockwise direction in Fig. 3B). Further, the notch portion 31h is provided with an end portion (second end portion) 31j with which the protrusion portion (first protrusion portion) 33f formed in the shaft portion 33a of the insulation spacer 33 comes into contact when the first forceps piece 31a rotates in a second direction opposite to the first direction (a direction in which the grip portion of the first forceps piece 31a moves away from the grip portion of the second forceps piece 31b, a clockwise direction in Fig. 3B) and the end portion (second end portion) 31j is provided as the other end facing the first end portion 31i in the notch portion 31h. A circumferential distance space of the notch portion 31h from the first end portion 31i to the second end portion 31j defines the rotation range of the first forceps piece 31a. Note that, in the present embodiment, when the first forceps piece 31a is opened by an angle of 55° with respect to the center axis of the outer sheath 11 as viewed from the rotation axis direction of the first forceps piece 31a, the protrusion portion 33f comes into contact with the second end portion 31j. Further, when the first forceps piece 31a becomes horizontal with respect to the center axis of the outer sheath 11, the protrusion portion 33f comes into contact with the first end portion 31i.

The inner peripheral surface of the shaft hole 31c of the second forceps piece 31b is provided with the notch portion (second notch portion) 31h which is widened from the axis center outward in the radial direction. The notch portion 31h is provided with the end portion (the third end portion) 31i with which the protrusion portion (second protrusion portion) 33f formed in the shaft portion 33b of the insulation spacer 33 comes into contact when the second forceps piece 31b rotates in the second direction (a direction in which the grip portion of the second forceps piece 31b moves close to the grip portion of the first forceps piece 31a, a clockwise direction in Fig. 3A). Further, the notch portion 31h is provided with the end portion (fourth end portion) 31j with which the protrusion portion (second protrusion portion) 33f formed in the shaft portion 33b of the insulation spacer 33 comes into contact when the second forceps piece 31b rotates in the first direction opposite to the second direction (a direction in which the grip portion of the second forceps piece 31b moves away from the grip portion of the first forceps piece 31a, a counter-clockwise direction in Fig. 3A) and the end portion (second end portion) 31j is provided as the other end facing the third end portion 31i of the notch portion 31h. A circumferential distance space of the notch portion 31h from the third end portion 31i to the fourth end portion 31j defines the rotation range of the second forceps piece 31b. Note that, in the present embodiment, when the second forceps piece 31b is opened by an angle of 55° with respect to the center axis of the outer sheath 11 as viewed from the rotation axis direction of the second forceps piece 31b, the protrusion portion 33f comes into contact with the fourth end portion 31j. Further, when the second forceps piece 31a becomes horizontal with respect to the center axis of the outer sheath 11, the protrusion portion 33f comes into contact with the third end portion 31i. Further, the pair of protrusion portions 33f protrudes outward in the radial direction of the rotation shaft (X axis) of the shaft portion 33a and is formed at the same circumferential position of the shaft portion 33a in the present embodiment. However, the pair of protrusions may not be formed at the same position.

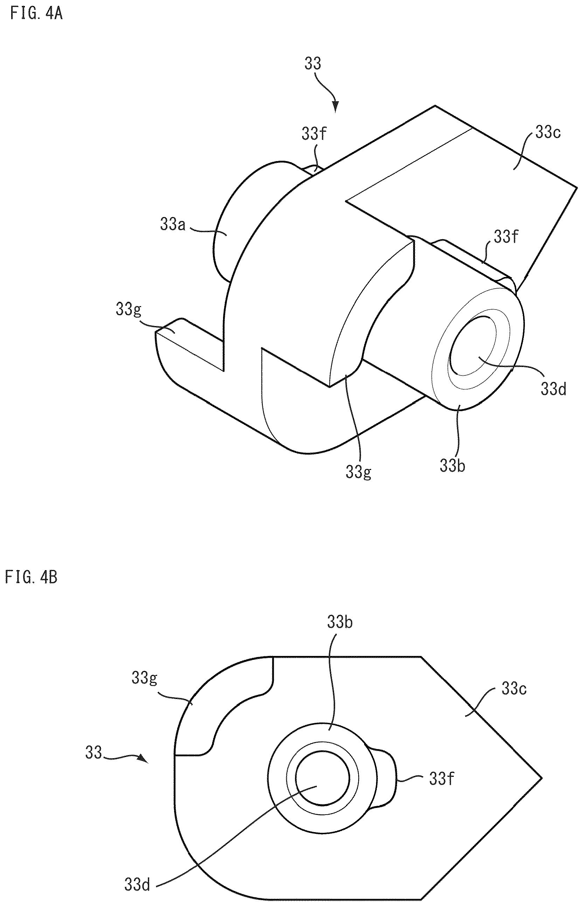

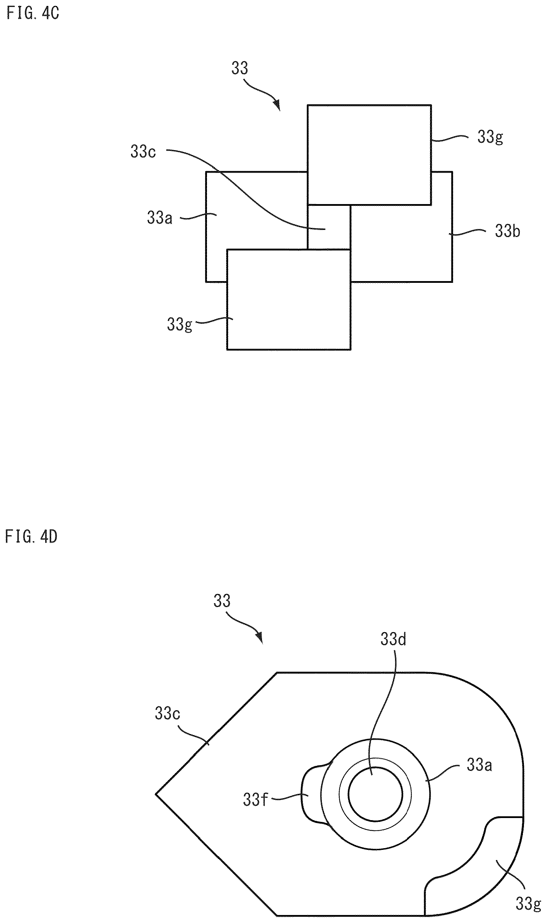

The insulation spacer 33 is, as illustrated in Figs. 4A to 4D, a member that rotates (turns) the first forceps piece 31a and the second forceps piece 31b while axially supporting them in an electrically insulated state. The insulation spacer 33 mainly includes an approximately plate-shaped spacer portion 33c, an approximately cylindrical shaft portion 33a which protrudes toward one surface of the spacer portion 33c, and an approximately cylindrical shaft portion 33b which protrudes toward the other surface of the spacer portion 33c. The shaft portion 33a and the shaft portion 33b are symmetrical to each other and are coaxially disposed. Through-holes 33d at the center portions of the shaft portion 33a and the shaft portion 33b communicate with each other to penetrate the spacer portion 33c.

The outer diameter of the shaft portion 33a is set to a value slightly smaller than the inner diameter of the shaft hole 31c of the first forceps piece 31a so as to be rotatably inserted into the shaft hole 31c of the first forceps piece 31a. The dimension (height) of the axial direction of each of the shaft portions 33a and 33b is set so that the front end portion of the shaft portion 33a is fitted to the fitting hole 32e of the arm portion 32a of the resin frame 32 and the front end portion of the shaft portion 33b is fitted to the fitting hole 32e of the arm portion 32b of the resin frame 32.

Further, the shaft portion 33a of the present embodiment includes the protrusion portion 33f which protrudes outward in the radial direction in the cross-section orthogonal to the axis of the shaft portion 33a. The protrusion portion 33f is formed to be movably disposed inside the notch portion 31h formed in the shaft hole 31c when the shaft portion 33a is inserted into the shaft hole 31c of the first forceps piece 31a. The dimension (height) of the axial direction of the protrusion portion 33f is set so that the protrusion portion comes into contact with the inner surface of the arm portion 32a when the front end portion of the shaft portion 33a is fitted to the fitting hole 32e of the arm portion 32a of the resin frame 32 and the front end portion of the shaft portion 33b is fitted to the fitting hole 32e of the arm portion 32b of the resin frame 32. That is, the protrusion portion 33f is set to a height in which the protrusion portion does not reach the fitting hole 32e.

Since the shaft portion 33b has substantially the same configuration as that of the shaft portion 33a except that the shaft portion is disposed to be plane-symmetrical to the above-described shaft portion 33a, a description thereof will be omitted.

Note that, in the present embodiment, the protrusion portion 33f is integrally formed with the shaft portions 33a and 33b from the base end near the spacer portion 33c to the front end coming into contact with the inner surfaces of the arm portions 32a and 32b along the shaft portions 33a and 33b, but may not be essentially formed to reach the spacer portion 33c and also may not come into contact with the inner surfaces of the arm portions 32a and 32b. Further, when the protrusion portion 33f is integrally formed along the shaft portions 33a and 33b, the protrusion portion 33f is reinforced by the shaft portions 33a and 33b. Therefore, this configuration is advantageous in terms of strength, but is not be essentially required. For example, the protrusion portion 33f may protrude from the spacer portion 33c to be independent from the shaft portions 33a and 33b and may be provided in approximately parallel to the shaft portions 33a and 33b. That is, a gap may be formed between the protrusion portion 33f and the shaft portions 33a and 33b.

The spacer portion 33c is integrally provided with a support protrusion 33g which protrudes toward one surface thereof and a support protrusion 33g which protrudes toward the other surface thereof. The dimension (height) of the axial direction of the support protrusion 33g is set so that the support protrusion comes into contact with the inner surfaces of the arm portions 32a and 32b when the front end portion of the shaft portion 33a is fitted to the fitting hole 32e of the arm portion 32a of the resin frame 32 and the front end portion of the shaft portion 33b is fitted to the fitting hole 32e of the arm portion 32b of the resin frame 32. That is, the dimension is set to be the same as the dimension of the axial direction of the protrusion portion 33f. The support protrusions 33g and 33g are used to define a gap between the pair of arm portions 32a and 32b of the resin frame 32 and are provided at a position in which the rotation of the corresponding forceps pieces 31a and 31b is not disturbed at a part of the edge portion of the spacer portion 33c.

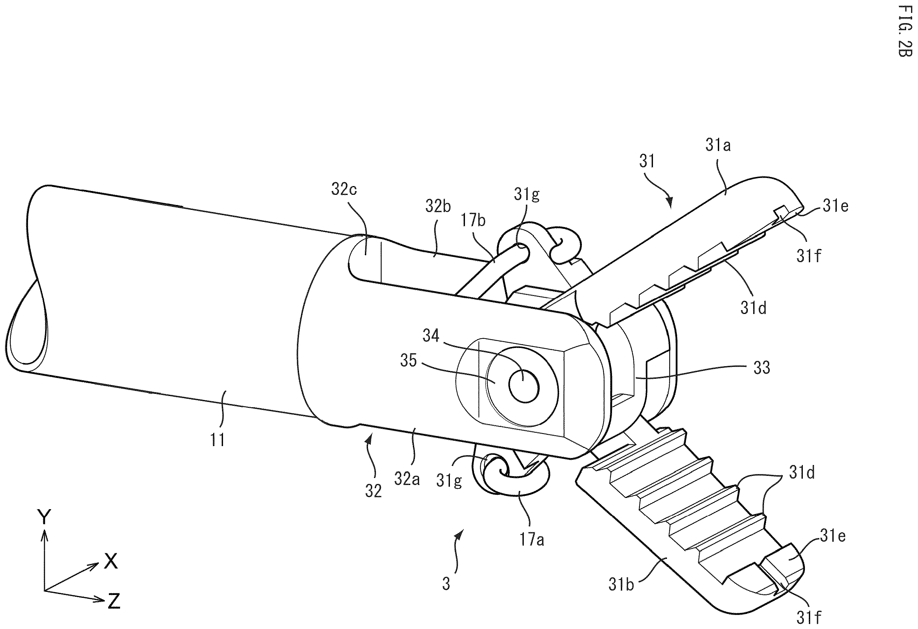

The first forceps piece 31a is axially supported by the insulation spacer 33 to be rotatable while the shaft portion 33a of the insulation spacer 33 is inserted into the shaft hole 31c and the second forceps piece 31b is axially supported by the insulation spacer 33 to be rotatable while the shaft portion 33b of the insulation spacer 33 is inserted into the shaft hole 31c. In this state, the front end portion of the shaft portion 33a of the insulation spacer 33 is fitted to the fitting hole 32e of the arm portion 32a and the front end portion of the shaft portion 33b of the insulation spacer 33 is fitted to the fitting hole 32e of the arm portion 32b while the pair of arm portions 32a and 32b of the resin frame 32 is slightly elastically deformed outward. Next, as illustrated in Fig. 2C, the kingpin 34 is inserted through the center through-holes 33d of the shaft portions 33a and 33b of the insulation spacer 33 and a washer 35 is fitted and fixed to the front end of the kingpin 34 from the opposite side as illustrated in Fig. 2D. Accordingly, as illustrated in Fig. 2A or 2B, the first forceps piece 31a and the second forceps piece 31b which are axially supported by the insulation spacer 33 to be rotatable are supported between the pair of arm portions 32a and 32b.

According to the present embodiment, the opening and closing angle ranges of the first forceps piece 31a and the second forceps piece 31b are defined by the end portions 31i and 31j of the notch portion 31h formed in the shaft hole 31c of the first forceps piece 31a and the protrusion portion 33f formed in the shaft portion 33a of the insulation spacer 33. Further, the opening and closing angle range of the second forceps piece 31b is defined by the end portions 31i and 31j of the notch portion 31h formed in the shaft hole 31c of the second forceps piece 31b and the protrusion portion 33f formed in the shaft portion 33b of the insulation spacer 33. When the opening and closing angles in a direction in which the first forceps piece 31a and the second forceps piece 31b are opened are defined, it is possible to solve a problem in which the forceps pieces 31a and 31b are opened too much and a portion to be clamped between the forceps pieces 31a and 31b is difficult to be targeted. Further, as in the embodiment, when the opening and closing angle is defined so that the forceps pieces 31a and 31b do not rotate in the closing direction at an angle equal to or larger than an angle in which each of the forceps pieces 31a and 31b becomes horizontal with respect to the center axis of the outer sheath 11, it is possible to prevent a problem in which one forceps piece 31a (31b) press-inserts the other forceps piece 31b (31a) so that the forceps portion 31 moves in a swinging manner and hence to stabilize the movement of the forceps portion 31 when the forceps portion 31 is pressed into the lumen or the like of the endoscope. In the related art, the opening and closing angle range of the forceps piece is defined in such a manner that a circular-arc groove is formed in a side surface of a spacer portion of an insulation spacer and a protrusion portion is provided in a side surface of an electrode to be loosely fitted to the groove. However, in the related art, only a relatively small protrusion portion can be formed in structure and strength cannot be sufficiently ensured.

In contrast, according to the structure of the present embodiment, it is possible to form the protrusion portion 33f to be relatively large outward in the radial direction and to secure sufficient strength. Further, in the related art, there is a case in which a protrusion portion is separated from a groove (the protrusion portions gets on a portion other than the groove), but such a problem can be prevented. Further, for example, when cutting is used to form the protrusion on the side surface (plane) of the forceps piece, there is a need to cut the other portion while remaining the protrusion portion. Accordingly, much effort and cost are necessary for the processing, but in the structure of the present embodiment, the protrusion portion 33f can be integrally molded with the insulation spacer 33 by, for example, resin molding. As a result, effort and cost for the processing can be reduced.

Note that, in the above-described first embodiment, the protrusion portion 33f is formed so that the protrusion portion 33f comes into contact with both end portions 31i and 31j of the notch portion 31h in response to the rotation of the forceps pieces 31a and 31b and is formed to define the opening and closing angles in both opening and closing directions of the forceps pieces 31a and 31b. However, the opening and closing angle may be defined by the protrusion portion 33f and the notch portion 31h only in any one of opening and closing direction and the opening and closing angle may not be defined or may be defined by other members in the other opening and closing angle.

Further, in the above-described first embodiment, the protrusion portion 33f is formed in a part of the outer peripheral surfaces of the shaft portions 33a and 33b and the notch portion 31h is formed in a part of the inner peripheral surface of the shaft hole 31c, but these components may be provided at the opposite positions. For example, the notch portion 31h may be formed in a part of the outer peripheral surfaces of the shaft portions 33a and 33b and the protrusion portion 33f may be formed in a part of the inner peripheral surface of the shaft hole 31c.

### Second Embodiment

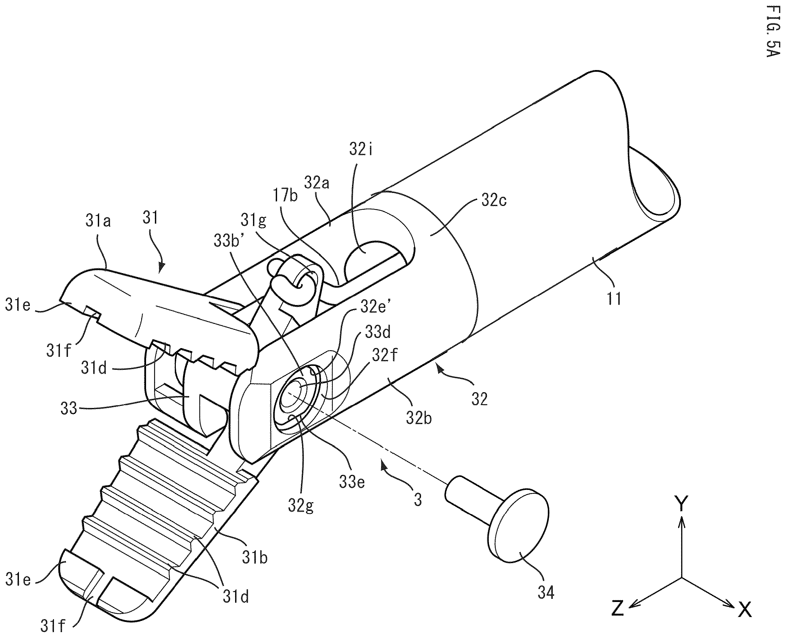

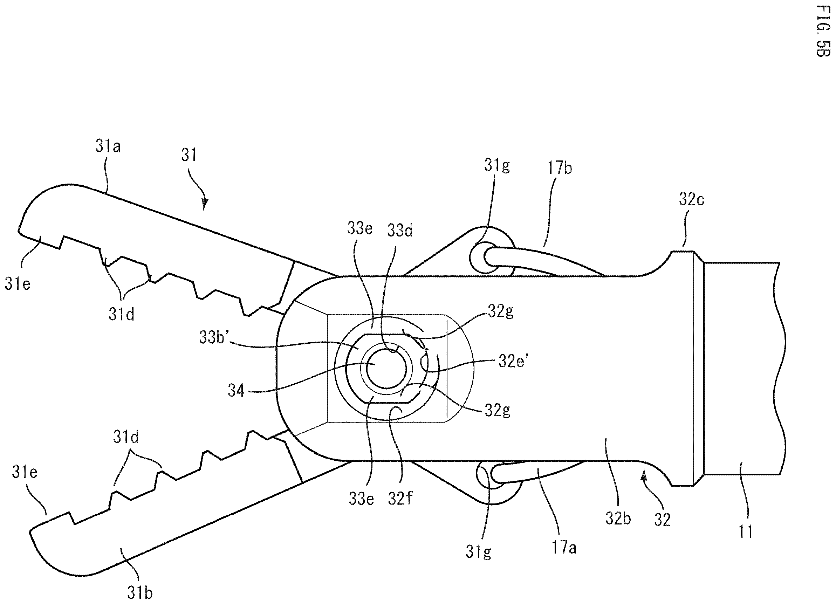

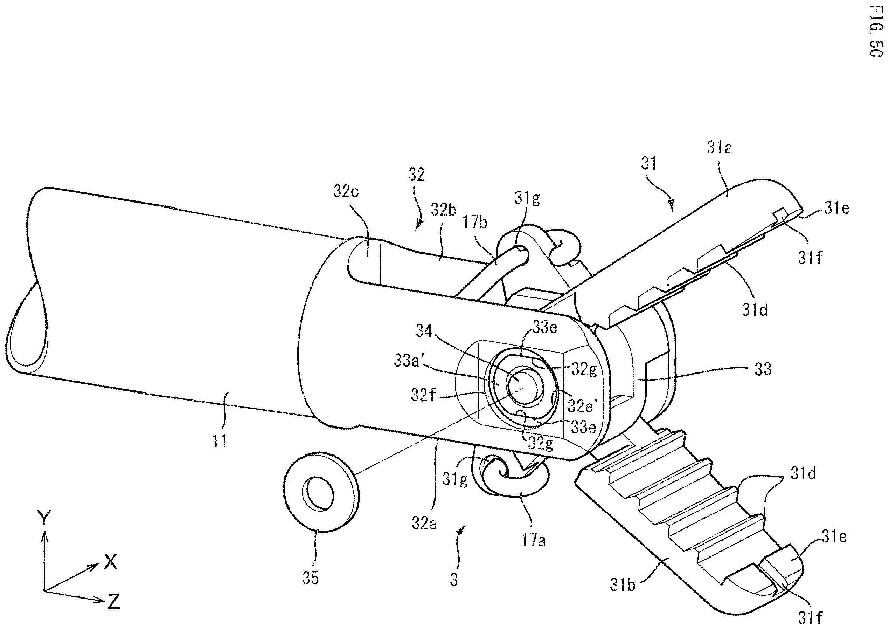

Hereinafter, a bipolar hemostat as a forceps type treatment instrument according to a second embodiment of the present invention will be described in detail with reference to Figs. 5A to 5C, 6A, 6B, and 7A to 7D. The same numerals will be given to the components having substantially the same configurations as those of the above-described first embodiment, a part of a description thereof will be omitted, and a difference will be mainly described.

In the above-described first embodiment, the shape (cross-sectional shape) of each of the fitting holes 32e and 32e of the arm portions 32a and 32b of the resin frame 32 was an approximately circular shape. In contrast, in the second embodiment, as illustrated in Figs. 5A to 5C, the shape (cross-sectional shape) of each of fitting holes 32e' and 32e' of the arm portions 32a and 32b of the resin frame 32 is a roughly oval shape (a shape obtained by cutting a circle with a pair of centrosymmetric parallel lines) provided with the complementary portions 32g and 32g so that the front end portions of the shaft portions 33a' and 33b' of the insulation spacer 33 described later are fittable.

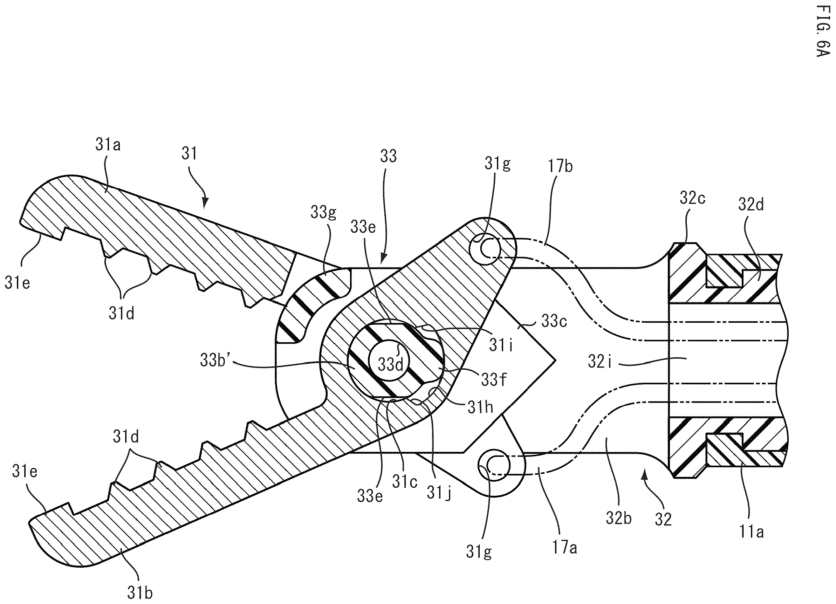

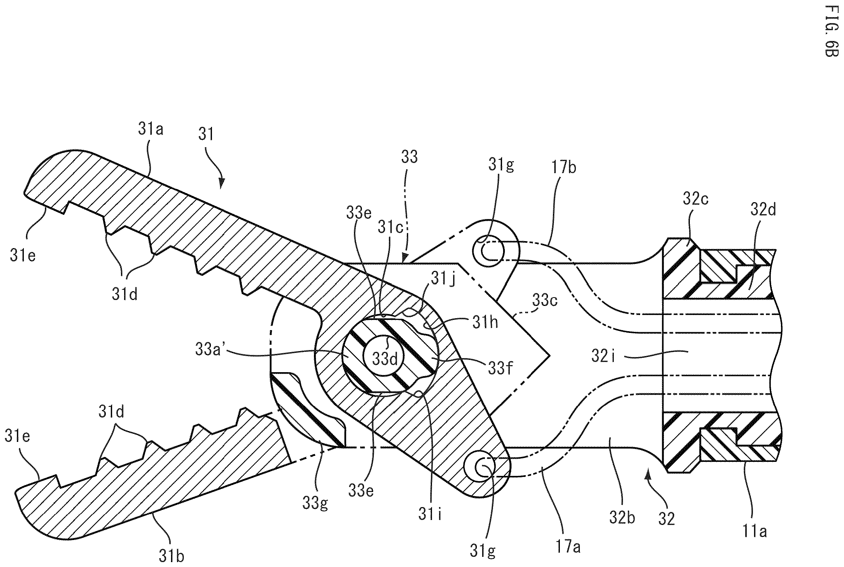

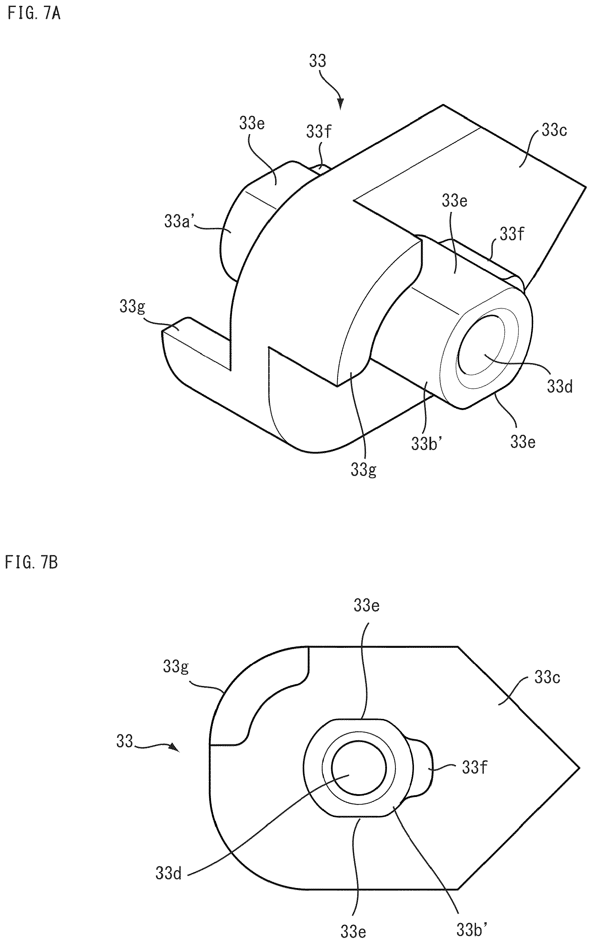

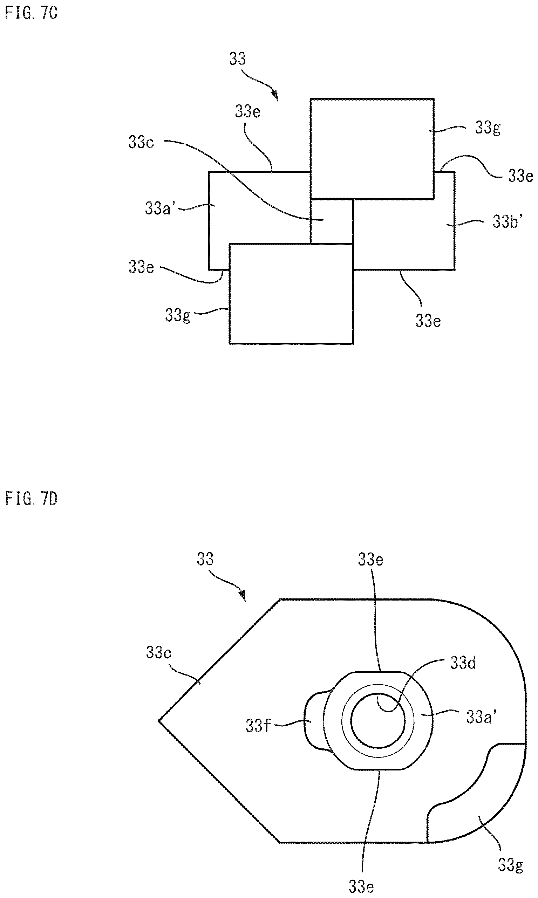

The shaft portion 33a' of the insulation spacer 33 is formed as illustrated in Figs. 6A, 6B, and 7A to 7D and the outer diameter thereof is set to a value slightly smaller than the inner diameter of the shaft hole 31c of the first forceps piece 31a so as to be rotatably inserted into the shaft hole 31c of the first forceps piece 31a. The shaft portion 33a' is provided with a pair of cutout portions 33e and 33e. The cutout portions 33e and 33e are formed as planes which are approximately parallel to the axis of the shaft portion 33a'. In the present embodiment, the plane forming one cutout portion 33e and the plane forming the other cutout portion 33e are set to be approximately parallel to each other. Thus, the cross-sectional shape of the shaft portion 33a' is a roughly oval shape (a shape obtained by cutting a circle with a pair of centrosymmetric parallel lines). However, planes forming the cutout portions 33e and 33e may not be parallel to each other as long as the planes are parallel to the axis of the shaft portion 33a'.

Since the shaft portion 33b' has substantially the same configuration as that of the shaft portion 33a' except that the shaft portion is plane-symmetrical to the above-described shaft portion 33a', a description thereof will be omitted.

In a state in which the front end portion of the shaft portion 33a' of the insulation spacer 33 is fitted to the fitting hole 32e' of the arm portion 32a of the resin frame 32 and the front end portion of the shaft portion 33b' of the insulation spacer 33 is fitted to the fitting hole 32e' of the arm portion 32b of the resin frame 32, the cutout portion 33e of each of the shaft portions 33a' and 33b' and the complementary portion 32g of the fitting hole 32e' of each of the arm portions 32a and 32b are fitted to engage with each other in the rotation direction. For this reason, since the rotation of the insulation spacer 33 with respect to the resin frame 32 is reliably prevented, it is possible to prevent a change in positional relationship between the resin frame 32 and the insulation spacer 33 regardless of the operation of opening and closing the first forceps piece 31a and the second forceps piece 31b and thus to stabilize the postures of the first forceps piece 31a and the second forceps piece 31b.

That is, when the insulation spacer 33 rotates with respect to the resin frame 32, there is concern that the direction of the distal end of the forceps portion 31 including the pair of forceps pieces 31a and 31b rotating with respect to the insulation spacer 33 to be opened and closed may be inclined with respect to the resin frame 32. In the forceps type treatment instrument of the second embodiment, since it is possible to reduce such concern, it is possible to provide the forceps type treatment instrument having excellent operability.

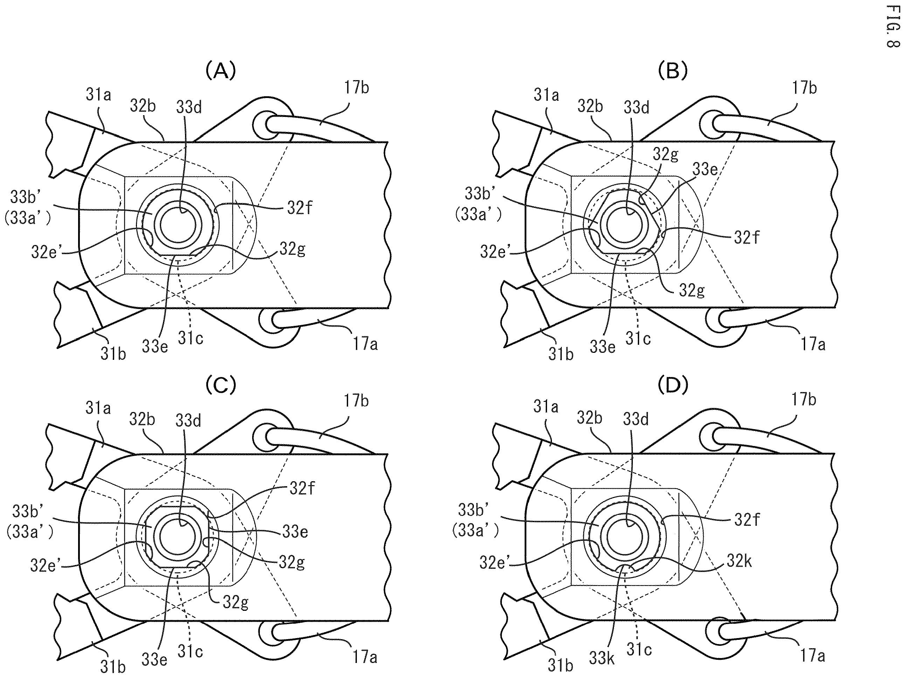

Next, a modified example of the complementary portion and the cutout portion for preventing the rotation of the insulation spacer 33 with respect to the resin frame 32 in the above-described second embodiment will be described with reference to Fig. 8. Further, the same numerals will be given to the components substantially having the same configurations as those of the above-described second embodiment and only a difference will be described.

In the above-described second embodiment, the pair of cutout portions 33e of each of the shaft portions 33a' and 33b' of the insulation spacer 33 is formed and the pair of complementary portions 32g of each of the fitting holes 32e' of the arm portions 32a and 32b of the resin frame 32 is formed to correspond to those components. However, the number of the cutout portions 33e and the complementary portions 32g is not limited to a pair (two), but may be single (one) as illustrated in Fig. 8(A), three as illustrated in Fig. 8(B), four as illustrated in Fig. 8(C), or four or more.

Further, as illustrated in Fig. 8(D), a concave cutout portion 33k of each of shaft portions 33a' and 33b' of the insulation spacer 33 may be formed and a convex complementary portion 32k having a shape of complementing the cutout portion 33k may be formed in the fitting hole 32e of each of the arm portions 32a and 32b of the resin frame 32 so as to correspond to those components. In this case, the shapes of the cutout portion 33k and the complementary portion 32k may be circular-arc shapes as illustrated in Fig. 8(D) or may be rectangular shapes or other shapes. The number of the cutout portions 33k and the complementary portions 32k may be also two or more.

Further, in the above-described second embodiment, the cutout portion 33e is provided at both of the shaft portion 33a'and 33b' of the insulation spacer 33, but may be provided only at one of them. In this case, the complementary portion 32g may be provided only at a portion corresponding to the fitting hole 32e' of each of the arm portions 32a and 32b of the resin frame 32.

Further, in the above-described second embodiment, the cutout portion 33e is provided across the entire portion (a portion from a base end near the spacer portion 33c to a front end) in the axial direction of the shaft portions 33a' and 33b' of the insulation spacer 33, but the cutout portion 33e may be provided only at the front end portion to be fitted to the fitting hole 32e' of each of the arm portions 32a and 32b.

### Third Embodiment

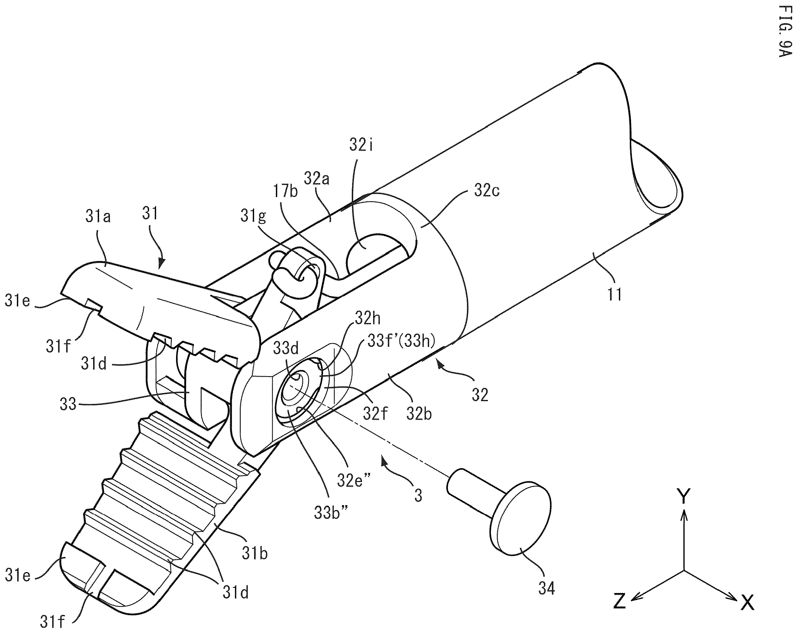

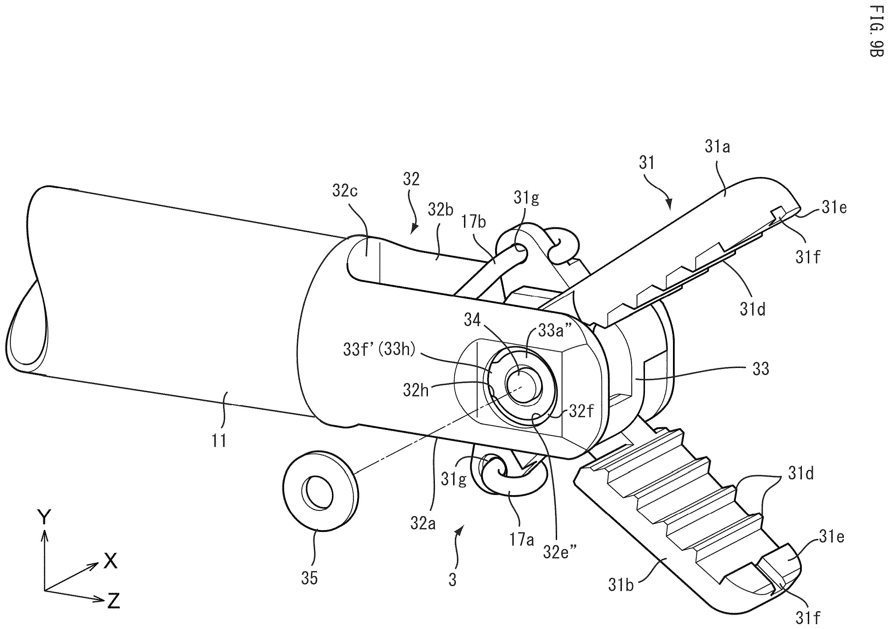

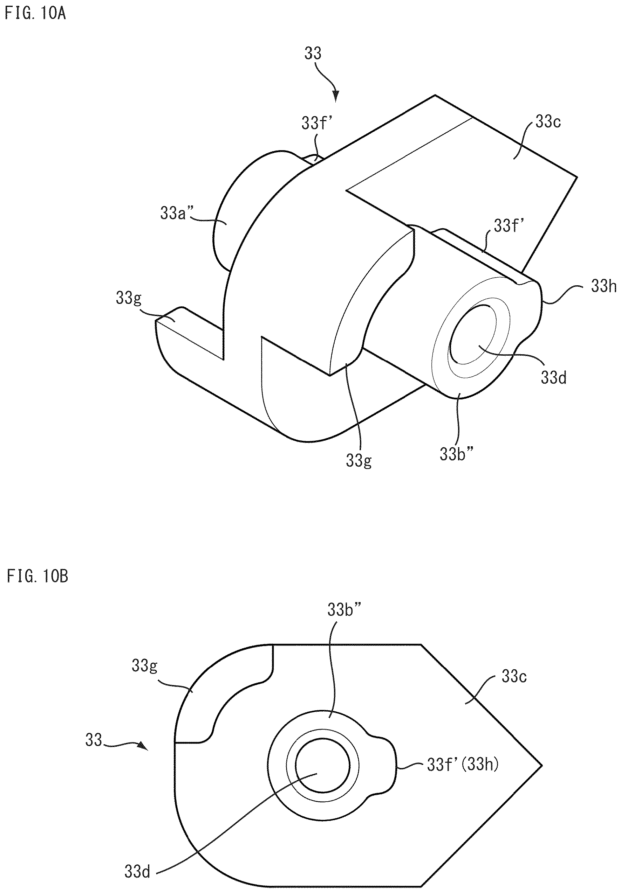

Next, a bipolar hemostat as a forceps type treatment instrument according to a third embodiment of the present invention will be described with reference to Figs. 9A, 9B, 10A, and 10B. The same numerals will be given to the components having substantially the same configurations as those of the above-described first embodiment and a difference will be described.

In the above-described first embodiment, as illustrated in Figs. 4A and 4B, the dimension (height) of the axial direction of each of the protrusion portions 33f of the shaft portions 33a and 33b of the insulation spacer 33 is set to be smaller (lower) than the dimension (height) of the axial direction of each of the shaft portions 33a and 33b of the insulation spacer 33 to have a step therebetween. In the third embodiment, as illustrated in Figs. 10A and 10B, the dimension (height) of the axial direction of each of protrusion portions 33f' of shaft portions 33a" and 33b" of the insulation spacer 33 is set to be the same as the dimension (height) of the axial direction of each of the shaft portions 33a" and 33b" of the insulation spacer 33. Further, as illustrated in Figs. 9A and 9B, the concave cutout portion 32h which is complemented by the front end portion 33h of the protrusion portion 33f' when the front end portions (the complementary portions) 33h of the protrusion portions 33f' are fitted to the fitting holes 32e" of the arm portions 32a and 32b of the resin frame 32 is formed in a part of the inner peripheral surface of the fitting hole 32e".

In a state in which the front end portion of the shaft portion 33a" of the insulation spacer 33 is fitted to the fitting hole 32e" of the arm portion 32a of the resin frame 32 and the front end portion of the shaft portion 33b" of the insulation spacer 33 is fitted to the fitting hole 32e" of the arm portion 32b of the resin frame 32, the front end portion (the complementary portion) 33h of the protrusion portion 33f' and the cutout portion 32h of the fitting hole 32e" of each of the arm portions 32a and 32b are fitted to engage with each other in the rotation direction. For this reason, since the rotation of the insulation spacer 33 with respect to the resin frame 32 is reliably prevented, it is possible to prevent a change in positional relationship between the resin frame 32 and the insulation spacer 33 regardless of the operation of opening and closing the first forceps piece 31a and the second forceps piece 31b and thus to stabilize the postures of the first forceps piece 31a and the second forceps piece 31b.

That is, when the insulation spacer 33 rotates with respect to the resin frame 32, there is concern that the direction of the distal end of the forceps portion 31 including the pair of forceps pieces 31a and 31b rotating with respect to the insulation spacer 33 to be opened and closed may be inclined with respect to the resin frame 32. In the forceps type treatment instrument of the third embodiment, since it is possible to reduce such concern, it is possible to provide the forceps type treatment instrument having excellent operability.

In the third embodiment, since the front end portions 33h of the protrusion portions 33f' of the shaft portions 33a" and 33b" of the insulation spacer 33 for defining the opening and closing angles of the pair of forceps pieces 31a and 31b serve as the complementary portions which complements the cutout portions 32h formed in the fitting holes 32e" of the arm portions 32a and 32b of the resin frame 32, it is possible to realize the same function as that of the second embodiment which prevents a change in posture (rotation) of the insulation spacer 33 with respect to the resin frame 32 in addition to a function of defining the opening and closing angles of the forceps pieces 31a and 31b of the above-described first embodiment. The others are the same as those of the above-described first embodiment.

The embodiments above are described to help the understanding of the present invention and are not described to limit the present invention. Therefore, each component disclosed in the embodiments above is intended to include all design changes and equivalents.

### EXPLANATIONS OF LETTERS OR NUMERALS

1 SHEATH PART
11 OUTER SHEATH
13 DRIVE WIRE
17a, 17b WIRE MEMBER
2 OPERATION PART
   21 BASE
   22 SLIDER
   23 END CAP
   24 PLUG
      24a, 24b ELECTRIC WIRE
3 TREATMENT PART
   31 FORCEPS PORTION
      31a, 31b ELECTRODE (FIRST FORCEPS PIECE, SECOND FORCEPS PIECE)
      31c SHAFT HOLE (FIRST SHAFT HOLE, SECOND SHAFT HOLE)
      31h NOTCH PORTION
      31i END PORTION (FIRST END PORTION, THIRD END PORTION)
      31j END PORTION (SECOND END PORTION, FOURTH END PORTION)
   32 RESIN FRAME (FORCEPS SUPPORT MEMBER)
      32a, 32b ARM PORTION (FIRST ARM PORTION, SECOND ARM PORTION)
      32c BASE END PORTION
      32e, 32e', 32e" FITTING HOLE (FIRST FITTING HOLE, SECOND FITTING HOLE)
      32h CUTOUT PORTION
   33 INSULATION SPACER
      33a, 33b, 33a', 33b', 33a", 33b" SHAFT PORTION (FIRST SHAFT PORTION, SECOND SHAFT PORTION)
      33c SPACER PORTION
      33d THROUGH-HOLE
      33f, 33f' PROTRUSION PORTION
      33g SUPPORT PROTRUSION
      33h FRONT END PORTION OF PROTRUSION PORTION (COMPLEMENTARY PORTION)

## Claims

1. A forceps type treatment instrument comprising:
a forceps support member (32) that includes a first arm portion (32a) and a second arm portion (32b) attached to a distal end of a sheath (11) and facing each other;
a first forceps piece (31a) and a second forceps piece (31b) which are interposed between the first arm portion (32a) and the second arm portion (32b) of the forceps support member (32); and
a spacer member (33) that rotatably supports each of the first forceps piece (31a) and the second forceps piece (31b),
wherein the first forceps piece (31a) includes a first shaft hole (31c),
wherein the second forceps piece (31b) includes a second shaft hole (31c),
wherein the spacer member (33) includes a first shaft portion (33a,33a') rotatably inserted into the first shaft hole (31c) and a second shaft portion (33b,33b') rotatably inserted into the second shaft hole (31c),
**characterized in that**
the spacer member (33) is provided with one of a first protrusion portion (33f) or a first notch portion (31h) and one of a second protrusion portion (33f) or a second notch portion (31h),
wherein an inner peripheral surface of the first shaft hole (31c) is provided with the other of the first protrusion portion (33f) or the first notch portion (31h),
wherein an inner peripheral surface of the second shaft hole (31c) is provided with the other of the second protrusion portion (33f) or the second notch portion (31h),
wherein the first notch portion (31h) is provided with a first end portion (31i) with which the first protrusion portion (33f) comes into contact when the first forceps piece (31a) is rotated in a first direction and a second end portion (31j) which is the other end facing the first end portion (31i), and
wherein the second notch portion (31h) is provided with a third end portion (31i) with which the second protrusion portion (33f) comes into contact when the second forceps piece (31b) is rotated in a second direction opposite to the first direction and a fourth end portion (31j) which is the other end facing the third end portion (31i).

2. The forceps type treatment instrument according to claim 1,
wherein the first protrusion portion (33f) comes into contact with the second end portion (31j) when the first forceps piece (31a) is rotated in the second direction, and
wherein the second protrusion portion (33f) comes into contact with the fourth end portion (31j) when the second forceps piece (31b) is rotated in the first direction.

3. The forceps type treatment instrument according to claim 1 or 2,
wherein the first protrusion portion (33f) is provided so as to protrude from an outer surface of the first shaft portion (33a), and
wherein the second protrusion portion (33f) is provided so as to protrude from an outer surface of the second shaft portion (33b).

4. A forceps type treatment instrument according to any one of claims 1 to 3,
wherein the first arm portion (32a) includes a first fitting hole (32e') into which a front end portion of the first shaft portion (33a') is fitted,
wherein the second arm portion (32b) includes a second fitting hole (32e') into which a front end portion of the second shaft portion (33b') is fitted,
wherein a cutout portion (33e) is provided in at least one of the first fitting hole (32e') and the front end portion of the first shaft portion (33a'), and
wherein a complementary portion (32g) which complements the cutout portion (33e) is provided in at least the other one of the first fitting hole (32e') and the front end portion of the first shaft portion (33a').

5. The forceps type treatment instrument according to claim 4,
wherein a cutout portion (33e) is provided in at least one of the second fitting hole (32e') and the front end portion of the second shaft portion (33b'), and
wherein a complementary portion (32g) which complements the cutout portion (33e) is provided in at least the other one of the second fitting hole (32e')and the front end portion of the second shaft portion (33b').

6. The forceps type treatment instrument according to claim 4 or 5,
wherein a cross-sectional shape orthogonal to an axis of at least the first fitting hole (32e') and the front end portion of the first shaft portion (33a') is a shape in which at least a part of a circular shape is cutting out.

7. The forceps type treatment instrument according to any one of claims 1 to 6,
wherein an outer peripheral surface of the first shaft portion (33a) is provided with the first protrusion portion (33f),
wherein an inner peripheral surface of the first shaft hole (31c) is provided with the first notch portion (31h),
wherein an outer peripheral surface of the second shaft portion (33b) is provided with the second protrusion portion (33f),
wherein an inner peripheral surface of the second shaft hole (31c) is provided with the second notch portion (31h)
wherein the first notch portion (31h) is provided with the first end portion (31i) with which the first protrusion portion (33f) comes into contact when the first forceps piece (31a) rotates in the first direction and the second end portion (31j) into which the first protrusion portion (31f) comes into contact when the first forceps piece (31a) rotates in the second direction opposite to the first direction, and
wherein the second notch portion (31h) is provided with the third end portion (31i) into which the second protrusion portion (33f) comes into contact when the second forceps piece (31b) rotates in the second direction and the fourth end portion (31j) into which the second protrusion portion (33f) comes into contact when the second forceps piece (31b) rotates in the first direction.

8. The forceps type treatment instrument according to any one of claims 1,2 or 4 to 6,
wherein an inner peripheral surface of the first shaft hole (31c) is provided with the first protrusion portion (33f)
wherein an outer peripheral surface of the first shaft portion (33a) is provided with the first notch portion (31h),
wherein an inner peripheral surface of the second shaft hole (31c) is provided with the second protrusion portion (33f),
wherein an outer peripheral surface of the second shaft portion (33b) is provided with the second notch portion (31h),
wherein the first notch portion (31h) is provided with the first end portion (31i) with which the first protrusion portion (33f) comes into contact when the first forceps piece (31a) rotates in the first direction and the second end portion (31j) into which the first protrusion portion (33f) comes into contact when the first forceps piece (31a) rotates in the second direction opposite to the first direction, and
wherein the second notch portion (31h) is provided with the third end portion (31i) into which the second protrusion portion (33f) comes into contact when the second forceps piece (31b) rotates in the second direction and the fourth end portion (31j) into which the second protrusion portion (33f) comes into contact when the second forceps piece (31b) rotates in the first direction.

## Patentansprüche

1. Ein zangenartiges Behandlungsinstrument, umfassend:
ein Zangenhalterungselement (32), das einen ersten Armabschnitt (32a) und einen zweiten Armabschnitt (32b) enthält, die an einem distalen Ende einer Hülle (11) angebracht sind und einander gegenüberliegen;
ein erstes Zangenteil (31a) und ein zweites Zangenteil (31b), die zwischen dem ersten Armabschnitt (32a) und dem zweiten Armabschnitt (32b) des Zangenhalterungselements (32)
angeordnet sind; und
ein Abstandselement (33), das sowohl das erste Zangenteil (31a) als auch das zweite Zangenteil (31b) drehbar hält,
wobei das erste Zangenteil (31a) ein erstes Schaftloch (31c) aufweist,
wobei das zweite Zangenteil (31b) ein zweites Schaftloch (31c) aufweist,
wobei das Abstandselement (33) einen ersten Schaftabschnitt (33a, 33a'), der drehbar in das erste Schaftloch (31c) eingesetzt ist, und einen zweiten Schaftabschnitt (33b, 33b'), der drehbar in das zweite Schaftloch (31c) eingesetzt ist, enthält,
**dadurch gekennzeichnet, dass**
das Abstandselement (33) mit einem ersten Vorsprungsabschnitt (33f) oder einem ersten Nutabschnitt (31h) und einem zweiten Vorsprungsabschnitt (33f) oder einem zweiten Nutabschnitt (31h) versehen ist,
wobei eine innere Umfangsfläche des ersten Schaftlochs (31c) entweder mit dem ersten Vorsprungsabschnitt (33f) oder dem ersten Nutabschnitt (31h) versehen ist,
wobei eine innere Umfangsfläche des zweiten Schaftlochs (31c) entweder mit dem zweiten Vorsprungsabschnitt (33f) oder dem zweiten Nutabschnitt (31h) versehen ist,
wobei der erste Nutabschnitt (31h) mit einem ersten Endabschnitt (31i), mit dem der erste Vorsprungsabschnitt (33f) in Kontakt kommt, wenn das erste Zangenteil (31a) in eine erste Richtung gedreht wird, und einem zweiten Endabschnitt (31j), welcher das andere Ende ist, das dem ersten Endabschnitt (31i) gegenüberliegt, versehen ist, und
wobei der zweite Nutabschnitt (31h) mit einem dritten Endabschnitt (31i), mit dem der zweite Vorsprungsabschnitt (33f) in Kontakt kommt, wenn das zweite Zangenteil (31b) in eine zweiten Richtung entgegengesetzt zur ersten Richtung gedreht wird, und mit einem vierten Endabschnitt (31j), welcher das andere Ende ist, das dem dritten Endabschnitt (31i) gegenüberliegt, versehen ist.

2. Das zangenartige Behandlungsinstrument nach Anspruch 1,
wobei der erste Vorsprungsabschnitt (33f) mit dem zweiten Endabschnitt (31j) in Kontakt kommt, wenn das erste Zangenteil (31a) in die zweite Richtung gedreht wird, und
wobei der zweite Vorsprungsabschnitt (33f) mit dem vierten Endabschnitt (31j) in Kontakt kommt, wenn das zweite Zangenteil (31b) in die erste Richtung gedreht wird.

3. Das zangenartige Behandlungsinstrument nach Anspruch 1 oder 2,
wobei der erste Vorsprungsabschnitt (33f) so vorgesehen ist, dass er von einer Außenfläche des ersten Schaftabschnitts (33a) vorsteht, und
wobei der zweite Vorsprungsabschnitt (33f) so vorgesehen ist, dass er von einer Außenfläche des zweiten Schaftabschnitts (33b) vorsteht.

4. Ein zangenartiges Behandlungsinstrument nach einem der Ansprüche 1 bis 3,
wobei der erste Armabschnitt (32a) ein erstes Passloch (32e') aufweist, in das ein vorderer Endabschnitt des ersten Schaftabschnitts (33a') eingepasst wird,
wobei der zweite Armabschnitt (32b) ein zweites Passloch (32e') aufweist, in das ein vorderer Endabschnitt des zweiten Schaftabschnitts (33b') eingepasst wird,
wobei ein Ausschnittabschnitt (33e) in mindestens einem von dem ersten Passloch (32e') und dem vorderen Endabschnitt des ersten Schaftabschnitts (33a') vorgesehen ist, und
wobei ein komplementärer Abschnitt (32g), welcher den Ausschnittabschnitt (33e) ergänzt, in mindestens dem anderen von dem ersten Passloch (32e') und dem vorderen Endabschnitt des ersten Schaftabschnitts (33a') vorgesehen ist.

5. Das zangenartige Behandlungsinstrument nach Anspruch 4,
wobei ein Ausschnittabschnitt (33e) in mindestens einem von dem zweiten Passloch (32e') und dem vorderen Endabschnitt des zweiten Schaftabschnitts (33b') vorgesehen ist, und
wobei ein komplementärer Abschnitt (32g), der den Ausschnittabschnitt (33e) ergänzt, in mindestens dem anderen von dem zweiten Passloch (32e') und dem vorderen Endabschnitt des zweiten Schaftabschnitts (33b') vorgesehen ist.

6. Das zangenartige Behandlungsinstrument nach Anspruch 4 oder 5,
wobei eine Querschnittsform rechtwinklig zu einer Achse zumindest des ersten Passlochs (32e') und des vorderen Endabschnitts des ersten Schaftabschnitts (33a') eine Form ist, in welcher zumindest ein Teil einer Kreisform ausgeschnitten ist.

7. Das zangenartige Behandlungsinstrument nach irgendeinem der Ansprüche 1 bis 6,
wobei eine äußere Umfangsfläche des ersten Schaftabschnitts (33a) mit dem ersten Vorsprungsabschnitt (33f) versehen ist,
wobei eine innere Umfangsfläche des ersten Schaftlochs (31c) mit dem ersten Nutabschnitt (31h) versehen ist,
wobei eine äußere Umfangsfläche des zweiten Schaftabschnitts (33b) mit dem zweiten Vorsprungsabschnitt (33f) versehen ist,
wobei eine innere Umfangsfläche des zweiten Schaftlochs (31c) mit dem zweiten Nutabschnitt (31h) versehen ist
wobei der erste Nutabschnitt (31h) mit dem ersten Endabschnitt (31i), mit dem der erste Vorsprungsabschnitt (33f) in Kontakt kommt, wenn sich das erste Zangenteil (31a) in die erste Richtung dreht, und dem zweiten Endabschnitt (31j), mit dem der erste Vorsprungsabschnitt (31f) in Kontakt kommt, wenn sich das erste Zangenteil (31a) in die zweite Richtung entgegengesetzt zur ersten Richtung dreht, versehen ist, und
wobei der zweite Nutabschnitt (31h) mit dem dritten Endabschnitt (31i), in den der zweite Vorsprungsabschnitt (33f) in Kontakt kommt, wenn sich das zweite Zangenteil (31b) in die zweite Richtung dreht, und dem vierten Endabschnitt (31j), in den der zweite Vorsprungsabschnitt (33f) in Kontakt kommt, wenn sich das zweite Zangenteil (31b) in die erste Richtung dreht, versehen ist.

8. Das zangenartige Behandlungsinstrument nach irgendeinem der Ansprüche 1, 2 oder 4 bis 6,
wobei eine innere Umfangsfläche des ersten Schaftlochs (31c) mit dem ersten Vorsprungsabschnitt (33f) versehen ist
wobei eine äußere Umfangsfläche des ersten Schaftabschnitts (33a) mit dem ersten Nutabschnitt (31h) versehen ist,
wobei eine innere Umfangsfläche des zweiten Schaftlochs (31c) mit dem zweiten Vorsprungsabschnitt (33f) versehen ist,
wobei eine äußere Umfangsfläche des zweiten Schaftabschnitts (33b) mit dem zweiten Nutabschnitt (31h) versehen ist,
wobei der erste Nutabschnitt (31h) mit dem ersten Endabschnitt (31i), mit dem der erste Vorsprungsabschnitt (33f) in Kontakt kommt, wenn sich das erste Zangenteil (31a) in die erste Richtung dreht, und dem zweiten Endabschnitt (31j), in den der erste Vorsprungsabschnitt (33f) in Kontakt kommt, wenn sich das erste Zangenteil (31a) in die zweite Richtung entgegengesetzt zur ersten Richtung dreht, versehen ist, und
wobei der zweite Nutabschnitt (31h) mit dem dritten Endabschnitt (31i), in den der zweite Vorsprungsabschnitt (33f) in Kontakt kommt, wenn sich das zweite Zangenteil (31b) in die zweite Richtung dreht, und dem vierten Endabschnitt (31j), in den der zweite Vorsprungsabschnitt (33f) in Kontakt kommt, wenn sich das zweite Zangenteil (31b) in die erste Richtung dreht, versehen ist.

## Revendications

1. Instrument de traitement de type pince comprenant :
un élément de support de pince (32) qui comprend une première partie de bras (32a) et une seconde partie de bras (32b) fixées à une extrémité distale d'une gaine (11) et se faisant face ;
une première pièce de pince (31a) et une seconde pièce de pince (31b) qui sont intercalées entre la première partie de bras (32a) et la seconde partie de bras (32b) de l'élément de support de pince (32) ; et
un élément d'espacement (33) qui supporte, en rotation, chacune parmi la première pièce de pince (31a) et la seconde pièce de pince (31b),
dans lequel la première pièce de pince (31a) comprend un premier trou de tige (31c),
dans lequel la seconde pièce de pince (31b) comprend un second trou de tige (31c),
dans lequel l'élément d'espacement (33) comprend une première partie de tige (33a, 33a') insérée, en rotation, dans le premier trou de tige (31c) et une seconde partie de tige (33b, 33b') insérée, en rotation, dans le second trou de tige (31c),
**caractérisé en ce que** :
l'élément d'espacement (33) est prévu avec l'une parmi une première partie de saillie (33f) ou une première partie d'encoche (31h) et l'une parmi une seconde partie de saillie (33f) ou une seconde partie d'encoche (31h),
dans lequel une surface périphérique interne du premier trou de tige (31c) est prévue avec l'autre parmi la première partie de saillie (33f) ou la première partie d'encoche (31h),
dans lequel une surface périphérique interne du second trou de tige (31c) est prévue avec l'autre parmi la seconde partie de saillie (33f) ou la seconde partie d'encoche (31h),
dans lequel la première partie d'encoche (31h) est prévue avec une première partie d'extrémité (31i) avec laquelle la première partie de saillie (33f) vient en contact lorsque la première pièce de pince (31a) est entraînée en rotation dans une première direction et une deuxième partie d'extrémité (31j) qui est l'autre extrémité faisant face à la première partie d'extrémité (31i), et
dans lequel la seconde partie d'encoche (31h) est prévue avec une troisième partie d'extrémité (31i) avec laquelle la seconde partie de saillie (33f) vient en contact lorsque la seconde pièce de pince (31b) est entraînée en rotation dans une seconde direction opposée à la première direction et une quatrième partie d'extrémité (31j) qui est l'autre extrémité faisant face à la troisième partie d'extrémité (31i).

2. Instrument de traitement de type pince selon la revendication 1,
dans lequel la première partie de saillie (33f) vient en contact avec la deuxième partie d'extrémité (31j) lorsque la première pièce de pince (31a) est entraînée en rotation dans la seconde direction, et
dans lequel la seconde partie de saillie (33f) vient en contact avec la quatrième partie d'extrémité (31j) lorsque la seconde pièce de pince (31b) est entraînée en rotation dans la première direction.

3. Instrument de traitement de type pince selon la revendication 1 ou 2,
dans lequel la première partie de saillie (33f) est prévue afin de faire saillie d'une surface externe de la première partie de tige (33a), et
dans lequel la seconde partie de saillie (33f) est prévue afin de faire saillie d'une surface externe de la seconde partie de tige (33b).

4. Instrument de traitement de type pince selon l'une quelconque des revendications 1 à 3,
dans lequel la première partie de bras (32a) comprend un premier trou de montage (32e') dans lequel une partie d'extrémité avant de la première partie de tige (33a') est montée,
dans lequel la seconde partie de bras (32b) comprend un second trou de montage (32e') dans lequel une partie d'extrémité avant de la seconde partie de tige (33b') est montée,
dans lequel une partie découpée (33e) est prévue dans au moins l'un parmi le premier trou de montage (32e') et la partie d'extrémité avant de la première partie de tige (33a'), et
dans lequel une partie complémentaire (32g) qui complète la partie découpée (33e) est prévue dans au moins l'autre parmi le premier trou de montage (32e') et la partie d'extrémité avant de la première partie de tige (33a').

5. Instrument de traitement de type pince selon la revendication 4,
dans lequel une partie découpée (33e) est prévue dans au moins l'un parmi le second trou de montage (32e') et la partie d'extrémité avant de la seconde partie de tige (33b'), et
dans lequel une partie complémentaire (32g) qui complète la partie découpée (33e) est prévue dans au moins l'autre parmi le second trou de montage (32e') et la partie d'extrémité avant de la seconde partie de tige (33b').

6. Instrument de traitement de type pince selon la revendication 4 ou 5,
dans lequel une forme de la section transversale orthogonale à un axe d'au moins le premier trou de montage (32e') et la partie d'extrémité avant de la première partie de tige (33a') est une forme dans laquelle au moins une partie d'une forme circulaire est découpée.

7. Instrument de traitement de type pince selon l'une quelconque des revendications 1 à 6,
dans lequel une surface périphérique externe de la première partie de tige (33a) est prévue avec la première partie de saillie (33f),
dans lequel une surface périphérique interne du premier trou de tige (31c) est prévue avec la première partie d'encoche (31h),
dans lequel une surface périphérique externe de la seconde partie de tige (33b) est prévue avec la seconde partie de saillie (33f),
dans lequel une surface périphérique interne du second trou de tige (31c) est prévue avec la seconde partie d'encoche (31h),
dans lequel la première partie d'encoche (31h) est prévue avec la première partie d'extrémité (31i) avec laquelle la première partie de saillie (33f) vient en contact lorsque la première pièce de pince (31a) tourne dans la première direction et la deuxième partie d'extrémité (31j) dans laquelle la première partie de saillie (31f) vient en contact lorsque la première pièce de pince (31a) tourne dans la seconde direction opposée à la première direction, et
dans lequel la seconde partie d'encoche (31h) est prévue avec la troisième partie d'extrémité (31i) dans laquelle la seconde partie de saillie (33f) vient en contact lorsque la seconde pièce de pince (31b) tourne dans la seconde direction et la quatrième partie d'extrémité (31j) dans laquelle la seconde partie de saillie (33f) vient en contact lorsque la seconde pièce de pince (31b) tourne dans la première direction.

8. Instrument de traitement de type pince selon l'une quelconque des revendications 1, 2 ou 4 à 6,
dans lequel une surface périphérique interne du premier trou de tige (31c) est prévue avec la première partie de saillie (33f),
dans lequel une surface périphérique externe de la première partie de tige (33a) est prévue avec la première partie d'encoche (31h),
dans lequel une surface périphérique interne du second trou de tige (31c) est prévue avec la seconde partie de saillie (33f),
dans lequel une surface périphérique externe de la seconde partie de tige (33b) est prévue avec la seconde partie d'encoche (31h),
dans lequel la première partie d'encoche (31h) est prévue avec la première partie d'extrémité (31i) avec laquelle la première partie de saillie (33f) vient en contact lorsque la première pièce de pince (31a) tourne dans la première direction et la deuxième partie d'extrémité (31j) dans laquelle la première partie de saillie (33f) vient en contact lorsque la première pièce de pince (31a) tourne dans la seconde direction opposée à la première direction, et
dans lequel la seconde partie d'encoche (31h) est prévue avec la troisième partie d'extrémité (31i) dans laquelle la seconde partie de saillie (33f) vient en contact lorsque la seconde pièce de pince (31b) tourne dans la seconde direction et la quatrième partie d'extrémité (31j) dans laquelle la seconde partie de saillie (33f) vient en contact lorsque la seconde pièce de pince (31b) tourne dans la première direction.
